# EUROPEAN PATENT SPECIFICATION

(11) **EP 1 562 520 B1**
(45) Date of publication and mention of the grant of the patent: **30.07.2008**
(21) Application number: 03811795.8
(22) Date of filing: 13.11.2003
(51) Int. Cl.: A61F 2/06

(54) **A HELICAL FORMATION FOR A CONDUIT**
SPIRALFÖRMIGER EINSATZ FÜR EIN ROHR
STRUCTURE HELICOIDALE DE CONDUIT

(30) Priority: 23.11.2002 GB 0227369
(43) Date of publication of application: 17.08.2005
(73) Proprietor: Tayside Flow Technologies Limited, Technology Park Dundee, DD2 1TY (GB)
(72) Inventor: HOUSTON, John Graeme, Perth, Tayside PH2 7AW (GB); HOOD, Robert Gordon, Tayside PH2 9AF (GB); STONEBRIDGE, Peter A., Tayside PH2 7AG (GB)
(74) Representative: Arends, William Gerrit
(86) International application number: PCT/GB2003/004917
(87) International publication number: WO 2004/047908

(56) References cited:
- EP-A- 1 254 645
- EP-A- 1 312 321
- EP-A- 1 314 406
- WO-A-03/103540
- AT-B- 134 543
- DE-A- 2 510 169
- DE-C- 597 472
- US-A1- 2002 179 166

## Description

The invention relates to a helical formation for a conduit.

A number of documents have proposed using helical formations in conduits to encourage a desired flow pattern of a fluid within the conduit. Such helical formations have been proposed for a wide variety of applications, including pipelines and blood flow tubing. The purpose of the helical formations is generally to generate spiral flow of the fluid within the conduit to reduce turbulence and dead spots within the conduit.

Although the use of helical formations has been proposed as beneficial to fluid flow in conduits by helping to generate spiral fluid flow patterns, there is little or no information on the physical characteristics of the helical formation that is required to create a suitable spiral flow pattern. Clearly, some designs of helical formations will be ineffective at creating spiral flow and other will not create a beneficial spiral flow. For example, helical formations having a high helix angle may tend to create turbulence rather than spiral flow due.

In accordance with a first aspect of the present invention, there is provided a helical formation for a conduit, the helical formation comprising an elongate member defining at least a portion of a helix, the elongate member comprising an inwardly extending portion, the inwardly extending portion extending along the length of the elongate member and extending inwardly from the internal side walls of the conduit for a distance equal to between 40% and 60% of the distance from the longitudinal axis of the conduit to an internal side wall.

The terms "helical", "helix" and "spiral" as used herein cover the mathematical definition of helical and any combination of the mathematical definitions of helical and spiral.

Preferably, the inwardly extending portion extends inwardly, for a distance equal to between 45% and 55%. Most preferably, the inwardly extending portion extends inwardly for a distance equal to substantially 50% of the distance from the longitudinal axis of the conduit to an internal side wall. Where the conduit has a circular cross-section, the distance is as a percentage of the radius of the conduit.

The helical formation may be in the form of an insert adapted to be inserted into the conduit, in use. The insert may be removably inserted or may be permanently inserted.

Alternatively, the helical formation may be an integral part of a side wall of the conduit. For example, the helical formation may be formed by a deformation of a portion of the side wall of the conduit.

In one example of the invention, the helical formation may be for use in blood flow tubing for the human or animal body. The tubing may be synthetic or natural blood flow tubing. For example, the tubing may be a graft. In another example, the conduit may be a stent for insertion into blood flow tubing in the human or animal body.

The helical formation may comprise two or more inwardly extending formations, arranged in side-by-side relationship extending along the length of the elongate member.

Examples of a helical formation in accordance with the invention will now be described with reference to the accompanying drawings, in which:
Figure 1 is a perspective view of a stent having a first example of a helical formation;
Figure 2 is a cross-sectional view of the stent;
Figure 3 is a perspective view of an arterial graft having a second example of a helical formation; and
Figure 4 is a cross-sectional view of the graft.

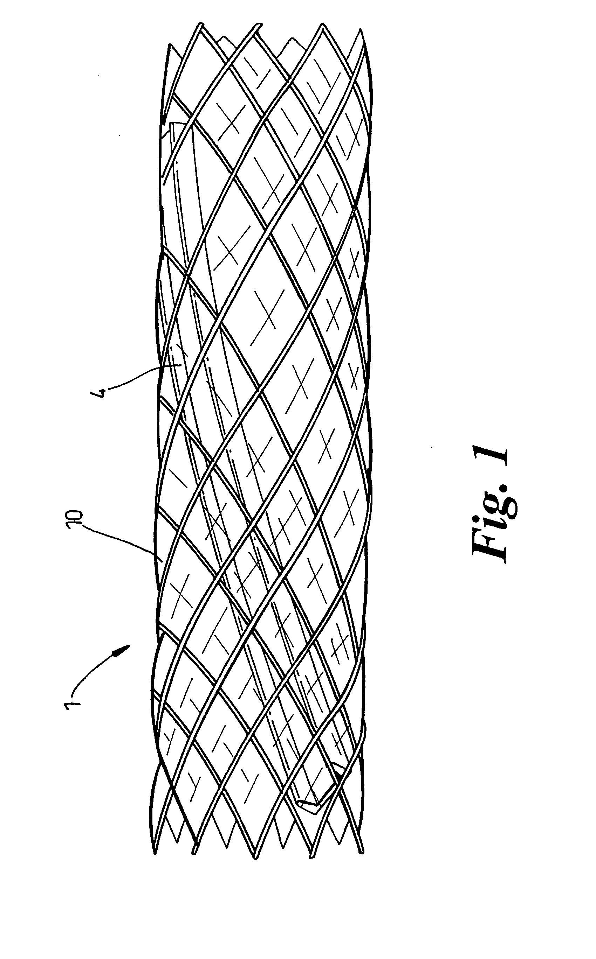

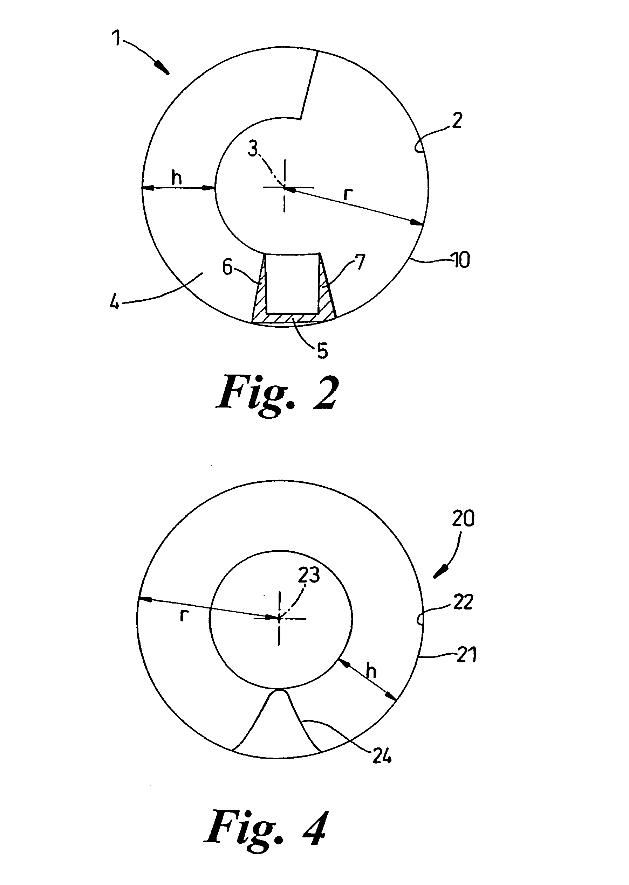

Figures 1 and 2 show a stent 1 having a body section 10 with an internal surface 2 and a longitudinal axis 3. The body section 10 has a circular cross-section. The body section 10 typically, has a mesh construction and may be, for example a metallic mesh. The distance r from the longitudinal axis 3 to the internal surface 2 is the internal radius of the stent 1. Within the stent 1 is a helical formation in the form of an insert 4. The insert 4 is helically shaped and defines a helix around the longitudinal axis 3. The insert 4 comprises a base portion 5 and two inwardly extending fins 6, 7, which extend along the length of the insert 4. The insert 4 is generally formed from a biocompatible material, such as polyurethane and may be melted onto the mesh structure of the stent 1 so that the material of the stent 1 is entrained within the material of the insert 4.

Each of the fins 6, 7 extend by a height h from the internal surface 2. The height h of the fins 6, 7 is equal to 50% of the internal radius, r. That is, h = r/2.

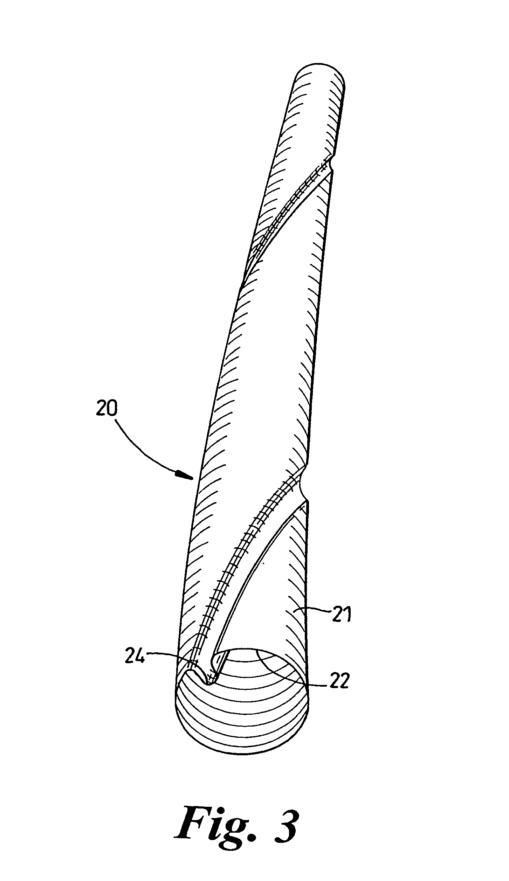

Figures 3 and 4 show an arterial graft 20 for blood flow tubing for use in the human or animal body. The graft 20 comprises a body section 21 having an internal surface 22 and a longitudinal axis 23. The graft 20 has internal radius r from the longitudinal axis to the internal surface 22. The body section 21 is typically formed from a biocompatible material, such as woven or knitted polyester. A helical formation 24 is formed by a deformation of the side wall of the body section 21. The helical formation 24 extends inwardly by a height h from the internal surface 22 and extends along the length of the graft 20 to define a helix around the longitudinal axis 23.

In the graft 20, the height, h, of the helical formation 24 equals 50% of the internal radius, r. That is, h = r/2 for the graft 20.

The inventors have found t hat a height h equal to r/2 (or 50% of the radius) i s particularly advantageous for generating spiral flow of blood within the stent 1 or the graft 20. They have also found that if the height h is too small, a negligible spiral flow pattern is produced by the insert 4 and the helical formation 24. In contrast, if the height h is too large relative to the internal radius r, the fins 6, 7 or the helical formation 24 tend to obturate the stent 1 or graft 20, respectively, and have a restrictive effect on flow.

While a height h = r/2 has been found to be produce a desired spiral flow pattern of blood in blood flow tubing, such as grafts and stents, the inventors have also found that other helical formation heights also have advantages in promoting spiral flow patterns. Therefore, the height h of the helical formation is typically, between 40% and 60% of the internal radius r and most preferably between 45% and 55%.

## Claims

1. A helical formation (4,24) for a conduit (1,20), the helical formation comprising an elongate member defining at least a portion of a helix, the elongate member comprising an inwardly extending portion, the inwardly extending portion extending along the length of the elongate member and extending inwardly from the internal side walls of the conduit, wherein the helical formulation is suitable for use in blood flow tubing or in a stent for insertion into blood flow tubing,
**characterized in that** the inwardly extending portion extends inwardly from the internal side walls of the conduit for a distance equal to between 40% and 60% of the distance from the longitudinal axis (3,23) of the conduit to an internal side wall (2,22).

2. A helical formation (4,24) according to claim 1. wherein the inwardly extending portion extends inwardly for a distance equal to between 45% and 55% of the distance from the longitudinal axis of the conduit to an internal side wall.

3. A helical formation (4,24) according to claim 2, wherein the inwardly extending portion extends inwardly for a distance equal to substantially 50% of the distance from the longitudinal axis of the conduit to an internal side wall.

4. A helical formation (4,24) according to any of the preceding claims, wherein the conduit (1,20) has a circular cross-section, and the distance that the inwardly extending portion extends inwardly is a percentage of the radius of the conduit.

5. A helical formation (4,24) according to any of the preceding claims, the helical formation comprising two or more inwardly extending formations (6,7), arranged in side-by-side relationship extending along the length of the elongate member.

6. A conduit (1,20) comprising a helical formation according to any one of the preceding claims, wherein the helical formation is mounted on a side wall (2,22) of the conduit.

7. A conduit (1,20) according to claim 6, wherein the helical formation (4,24) is in the form of an insert adapted to be inserted into the conduit.

8. A conduit (1,20) according to claim 7, wherein the insert is removable from the conduit.

9. A conduit (1,20) according to claim 6, wherein the helical formation (4,24) is an integral part of the side wall of the conduit.

10. A conduit (1,20) according to claim 9, wherein the helical formation (4,24) is formed by a deformation of a portion of the side wall (21) of the conduit.

11. A conduit (1,20) according to any one of claims 6 to 10, wherein the conduit comprises blood flow tubing for use in the human or animal body.

12. A conduit (1,20) according to claim 11, wherein the blood flow tubing comprises a vascular graft.

13. A conduit (1,20) according to any one of claims 6 to 11, wherein the conduit comprises a stent (1) for insertion into blood flow tubing in the human or animal body.

## Patentansprüche

1. Spiralförmiger Einsatz (4, 24) für ein Rohr (1, 20), wobei der spiralförmige Einsatz ein längliches Element aufweist, das mindestens einen Abschnitt einer Spirale definiert, wobei das längliche Element einen sich nach innen erstreckenden Abschnitt aufweist, wobei der sich nach innen erstreckende Abschnitt sich entlang der Länge des länglichen Elementes erstreckt und sich nach innen von den inneren Seitenwänden des Rohres erstreckt, wobei der spiralförmige Einsatz für eine Verwendung in einem Blutflussrohr oder in einem Stent für ein Einsetzen in das Blutflussrohr geeignet ist,
**dadurch gekennzeichnet, dass** der sich nach innen erstreckende Abschnitt sich nach innen von den inneren Seitenwänden des Rohres über eine Strecke erstreckt, die zwischen 40 % und 60 % der Strecke von der Längsachse (3, 23) des Rohres zu einer inneren Seitenwand (2, 22) beträgt.

2. Spiralförmiger Einsatz (4, 24) nach Anspruch 1, bei dem der sich nach innen erstreckende Abschnitt sich nach innen über eine Strecke erstreckt, die zwischen 45 % und 55 % der Strecke von der Längsachse des Rohres zu einer inneren Seitenwand beträgt.

3. Spiralförmiger Einsatz (4, 24) nach Anspruch 2, bei dem der sich nach innen erstreckende Abschnitt sich nach innen über eine Strecke erstreckt, die im Wesentlichen 50 % der Strecke von der Längsachse des Rohres zu einer inneren Seitenwand beträgt.

4. Spiralförmiger Einsatz (4, 24) nach einem der vorhergehenden Ansprüche, bei dem das Rohr (1, 20) einen kreisförmigen Querschnitt aufweist und die Strecke, über die sich der sich nach innen erstreckende Abschnitt nach innen erstreckt, ein Prozentwert des Radiusses des Rohres ist.

5. Spiralförmiger Einsatz (4, 24) nach einem der vorhergehenden Ansprüche, wobei der spiralförmige Einsatz zwei oder mehr sich nach innen erstreckende Einsätze (6, 7) aufweist, die in einer nebeneinanderliegenden Beziehung angeordnet sind, wobei sie sich entlang der Länge des länglichen Elementes erstrecken.

6. Rohr (1, 20), das einen spiralförmigen Einsatz nach einem der vorhergehenden Ansprüche aufweist, wobei der spiralförmige Einsatz an einer Seitenwand (2, 22) des Rohres montiert ist.

7. Rohr (1, 20) nach Anspruch 6, wobei der spiralförmige Einsatz (4, 24) in der Form eines Einsatzes vorliegt, der so ausgeführt ist, dass er in das Rohr eingesetzt wird.

8. Rohr (1, 20) nach Anspruch 7, wobei der Einsatz aus dem Rohr entfernt werden kann.

9. Rohr (1, 20) nach Anspruch 6, wobei der spiralförmige Einsatz (4, 24) ein integrierter Bestandteil der Seitenwand des Rohres ist.

10. Rohr (1, 20) nach Anpruch 9, wobei der spiralförmige Einsatz (4, 24) durch eine Verformung eines Abschnittes der Seitenwand (21) des Rohres gebildet wird.

11. Rohr (1, 20) nach einem der Ansprüche 6 bis 10, wobei das Rohr ein Blutflussrohr für eine Verwendung im menschlichen oder tierischen Körper aufweist.

12. Rohr (1, 20) nach Anspruch 11, bei dem das Blutflussrohr ein Gefäßtransplantat aufweist.

13. Rohr (1, 20) nach einem der Ansprüche 6 bis 11, wobei das Rohr einen Stent (1) für ein Einsetzen in das Blutflussrohr im menschlichen oder tierischen Körper aufweist.

## Revendications

1. Structure hélicoïdale (4, 24) pour un conduit (1, 20), la structure hélicoïdale comprenant un élément allongé définissant au moins une partie d'une hélice, l'élément allongé comprenant une partie s'étendant vers l'intérieur, la partie s'étendant vers l'intérieur s'étendant le long de la longueur de l'élément allongé et s'étendant vers l'intérieur à partir des parois latérales internes du conduit, la structure hélicoïdale se prêtant à une utilisant dans une tubulure d'écoulement de sang ou dans un stent destiné à être inséré dans une tubulure d'écoulement de sang ;
**caractérisée en ce que** la partie s'étendant vers l'intérieur s'étend vers l'intérieur à partir des parois latérales internes du conduit sur une distance représentant entre 40% et 60% de la distance entre l'axe longitudinal (3, 23) du conduit et une paroi latérale interne (2, 22).

2. Structure hélicoïdale (4, 24) selon la revendication 1, dans laquelle la partie s'étendant vers l'intérieur s'étend vers l'intérieur sur une distance représentant entre 45% et 55% de la distance entre l'axe longitudinal du conduit et une paroi latérale interne.

3. Structure hélicoïdale (4, 24) selon la revendication 2, dans laquelle la partie s'étendant vers l'intérieur s'étend vers l'intérieur sur une distance représentant environ 50% de la distance entre l'axe longitudinal du conduit et une paroi latérale interne.

4. Structure hélicoïdale (4, 24) selon l'une quelconque des revendications précédentes, dans laquelle le conduit (1, 20) a une section transversale circulaire, la distance d'extension vers l'intérieur de la partie s'étendant vers l'intérieur correspondant à un pourcentage du rayon du conduit.

5. Structure hélicoïdale (4, 24) selon l'une quelconque des revendications précédentes, la structure hélicoïdale comprenant deux ou plusieurs structures s'étendant vers l'intérieur (6, 7), agencées côte à côte et s'étendant le long de la longueur de l'élément allongé.

6. Conduit (1, 20), comprenant une structure hélicoïdale selon l'une quelconque des revendications précédentes, dans lequel la structure hélicoïdale est montée sur une paroi latérale (2, 22) du conduit.

7. Conduit (1, 20) selon la revendication 6, dans lequel la structure hélicoïdale (4, 24) a la forme d'un insert adapté pour être inséré dans le conduit.

8. Conduit (1, 20) selon la revendication 7, dans lequel l'insert peut être retiré du conduit.

9. Conduit (1, 20) selon la revendication 6, dans lequel la structure hélicoïdale (4, 24) fait partie intégrante de la paroi latérale du conduit.

10. Conduit (1, 20) selon la revendication 9, dans lequel la structure hélicoïdale (4, 24) est formée par une déformation d'une partie de la paroi latérale (21) du conduit.

11. Conduit (1, 20) selon l'une quelconque des revendications 6 à 10, dans lequel le conduit comprend une tubulure d'écoulement de sang destinée à être utilisée dans un corps humain ou dans le corps d'un animal.

12. Conduit (1, 20) selon la revendication 11, dans lequel la tubulure d'écoulement de sang comprend une greffe vasculaire.

13. Conduit (1, 20) selon l'une quelconque des revendications 6 à 11, dans lequel le conduit comprend un stent (1) destiné à être inséré dans une tubulure d'écoulement de sang dans le corps humain ou dans le corps d'un animal.
